# EUROPEAN PATENT SPECIFICATION

(11) **EP 2 907 913 B1**
(45) Date of publication and mention of the grant of the patent: **14.09.2016**
(21) Application number: 15154454.1
(22) Date of filing: 10.02.2015
(51) Int. Cl.: D06F 43/00, D06L 1/04

(54) **A perfuming element for garments usable in dry cleaning, and a relative dry-cleaning process**
Beduftungselement für Kleidungsstücke zur Verwendung in der chemischen Reinigung und zugehöriges chemisches Reinigungsverfahren
Élément parfumant pour des vêtements pouvant être utilisé dans le nettoyage à sec, et méthode de nettoyage à sec correspondant

(30) Priority: 12.02.2014 IT RE20140009
(43) Date of publication of application: 19.08.2015
(73) Proprietor: Sanflor S.r.l., 46100 Mantova (IT)
(72) Inventor: Bonizzi, Sergio, 46047 Porto Mantovano (Mantova) (IT)
(74) Representative: Corradini, Corrado

(56) References cited:
- EP-A2- 1 420 101
- WO-A1-01/53594
- WO-A1-01/94521
- WO-A1-97/32004
- FR-A1- 2 360 476
- US-A1- 2004 129 032

## Description

### TECHNICAL FIELD

The present invention concerns a perfuming element for clothing suitable for use in dry cleaning and a relative method of dry cleaning.

More in particular, the invention relates to a perfuming element for clothing suitable for use in dry cleaning processes using chemical solvents able to dissolve and remove grease stains from clothing and the relative dry cleaning process using the perfuming element.

### PRIOR ART

As is known, in the field of garment cleaning there are two main types of washing, washing with water, generally open-cycle, and dry cleaning, generally in a closed cycle.

The first type of cleaning involves the use of detergents, the composition of which also includes a certain dose of perfume that during washing in water transfers to the garment while it is being cleaned by effect of surfactants present in the detergent and the mechanical action exerted by the water.

While this first type of washing is particularly effective and economical for clothing stained with common dirt, it is not equally effective with spots of oil-based solutions, such as oil, grease or the like.

The second type of cleaning, however, which is particularly effective in the cleaning of oil-based stains, involves the insertion of the garment in a solvent bath, such as perchlorethylene, liquid carbon dioxide or cyclic siloxane compounds, which carry the oil substances in the solution and remove them from the garment, which emerges clean after drying the solvent off.

A drawback encountered in dry cleaning, however, is that solvents having a strong aromatic smell used for the removal of greasy stains permeate the garments with the odour, and it is not easy to perfume the garments once dried; this means that the aromatic odour is released from the garment also in stages following cleaning, such as during storage or ironing or the like.

Further, dry cleaning makes perfuming the garments impossible in a solvent bath, as instead happens during washing in water, since any perfuming substance, generally also composed of natural or synthetic essential oils, and therefore based on oily substances, having been inserted into a solvent bath would be washed away, as is indeed the case for greasy stains of oil or grease on clothing, leaving the garments substantially free of perfume and impregnated with the strongest and most persistent of the solvent odour. WO 01/53594 A1 describes devices for containment and treating or refreshening of fabric articles in a rotains clothes dryer. An aim of the present invention is to obviate the above-mentioned drawbacks of dry cleaning according to the known process, with a solution that is simple, rational and relatively inexpensive.

These aims are attained by the characteristics of the invention as reported in the independent claims 1 and 11. The dependent claims describe preferred and/or particularly advantageous aspects of the invention.

### DESCRIPTION OF THE INVENTION

In particular, the invention discloses a perfuming element for garments, usable in dry cleaning, based on solvents (for example organic solvents), which comprises a perfuming substance supported by a gradual-release support element of the perfuming substance.

With this solution, the solvent does not attack the perfuming substance; at least a part thereof is adsorbed on the support element and this can therefore perfume the garment being washed at the end of the cycle.

In an further aspect of the invention, the support element is made of a plastic material provided with micropores.

In this way, the perfuming substance can be adbsorbed internally of the cavities defined by the micropores, entering protected zones of the support element, which limit the chemical attack of the solvent on the perfuming substance and slow down the diffusion of the fragrance of the perfuming substance itself.

The support element is made of polyethylene in granule form.

Thanks to this solution, the adsorbent power of the support element is optimised, as is the release action of the perfuming substance thereof.

In fact, polyethylene is a substance exhibiting a thermal dilation in the range of temperatures in which garments are dried in the dry-cleaning cycle, for example between 50°C and 60°C.

Thus the granules of polyethylene, and therefore the micropores thereof, pass from a contracted configuration to an expanded configuration when the temperature of the granule exceeds a determined threshold value, for example when the temperature of the granule is substantially comprised between 50°C and 60°C.

When the polyethylene granules, and therefore the micropores, are in the contracted configuration, they strongly limit or prevent exit of the perfuming substances from the internal cavities of the granule.

This effect is also amplified by the surface tension between the perfuming - solvent interface.

Thus, during dry-cleaning of the clothes, in which the temperature is lower than 50°C and the garment is immersed in the solvent bath, the polyethylene granule in the contracted configuration strongly limits (slows down) or prevents at most the exit of the perfuming substance, which is therefore protected from the attack of the solvent.

When the polyethylene granules, and therefore the micropores, are in the expanded configuration, the perfuming substance is free to exit (by capillary action) from the internal cavities of the granule.

Thus when at the end of the dry-cleaning operation of the clothes a majority of the solvent has been removed from the washing drum and the machine proceeds to the drying of the clothes from the residual solvent remaining in the clothes, the temperature is raised for example to between 50°C and 60°C (for example for a substantially identical time to the washing time, i.e. about 25 minutes), the polyethylene granule expands to the expanded configuration, which can be released, in all its components (head, body and tail), and in a large quantity on the garments which therefore can be dry and perfumed to perfection.

In an aspect of the invention, the granulometry of the support element is substantially comprised between 0.2 mm and 1 cm.

The perfuming element, especially if granular, is advantageously contained internally of a porous bag, for example made of a non-woven fabric; in this way the perfuming element can be confined and made easy to handle while being in granular form and therefore with a high exchange capacity of the perfuming substance.

In an aspect of the invention, the perfuming substance comprises a mixture of essential oils comprising at least one from among citronellyl nitrile, allyl cyclohexylpropionate, aldehyde c14, allyl amyl glycolate, vertocitral and dynascone pure.

With this mixture, the persistence, intensity and pleasantness of the perfumed fragrance is optimized, which attributes remain in the garment after the dry-cleaning thereof.

Still more preferably, in an advantageous formulation the mixture of essential oils comprises citronellyl nitrile in quantities comprised between 8 and 15 per thousand with respect to the weight of the mixture, allyl cyclohexylpropionate in quantities comprised between 12 and 20 per thousand with respect to the weight of the mixture, aldehyde c14 in quantities comprised between 20 and 30 per thousand with respect to the weight of the mixture, allyl amyl glycolate in quantities comprised between 7 and 18 per thousand with respect to the weight of the mixture, vertocitral in quantities comprised between 12 and 17 per thousand with respect to the weight of the mixture and pure dynascone in quantities comprised between 4 and 10 per thousand with respect to the weight of the mixture.

In a further aspect of the invention, the perfuming substance and the support element constituting the perfuming element are in a weight ratio of substantially 1:1 (or close to that ratio).

A further aspect of the invention discloses a dry cleaning process of garments comprising steps of:
- locating at least a garment to be cleaned in a drum;
- introducing at least a liquid solvent into the cleaning drum;
- extracting the solvent from the cleaning drum and
- filtering the solvent that has come into contact with the garment;

According to the invention, the process comprises a step of adding, into the cleaning drum, together with the garment to be cleaned, at least a dosed quantity of a perfuming element of the garment as described above.

With this solution the garments subjected to dry cleaning can be perfumed in a way that is pleasant, persistent and simple to obtain.

The perfuming element is advantageously added in a dose comprised between 5 and 100 grams per each 10-20 kilograms of garments.

This dose has provided an excellent effectiveness of perfuming of the garments with a good price-quality ratio.

A further aspect the process of the invention comprises a step of distilling the solvent extracted from the cleaning drum and/or centrifuging and/or drying, for example by heating, the garment internally of the cleaning drum.

The heating internally of the washing drum can be done at a temperature comprised between 50° and 60°.

For example, the solvent is a chemical solvent able to dissolve and remove grease marks (oil, fat and the like) from garments; for example it is a chemical solvent selected from among a group including perchloroethylene, aliphatic hydrocarbons, liquid carbon dioxide and cyclic siloxane compounds.

### BRIEF DESCRIPTION OF THE DRAWINGS

Further characteristics and advantages of the invention will emerge from a reading of the description that follows, provided by way of non-limiting example, with the aid of the figures of the accompanying tables.
Figure 1 is a schematic view of a perfuming element according to the invention.
Figure 2 is a schematic view of a dry-cleaning machine.

### BEST WAY OF CARRYING OUT THE INVENTION

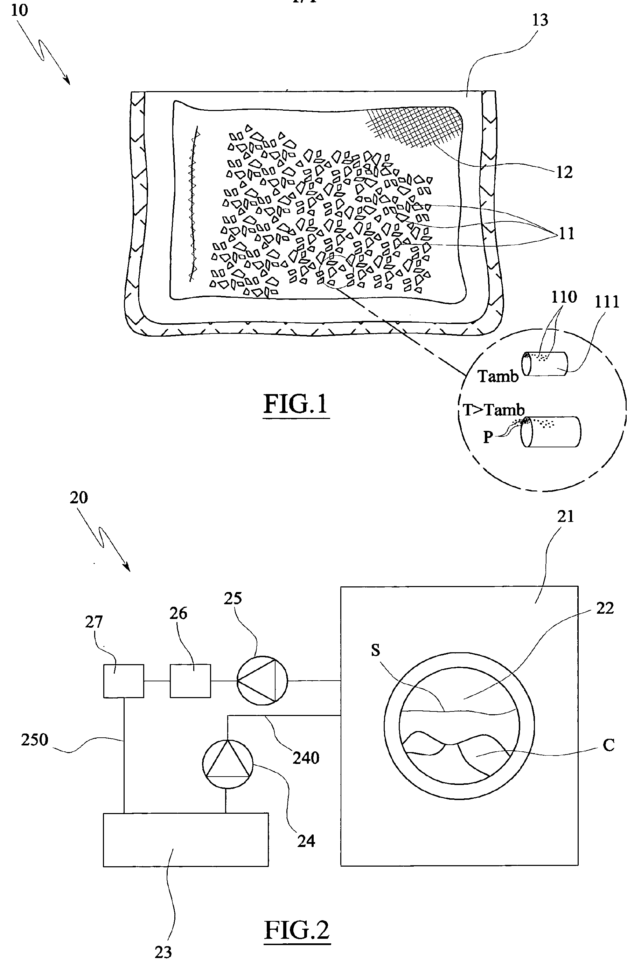

With particular reference to the figures, 10 denotes in its entirely a perfuming element suitable for perfuming garments (denoted in figure 2 by reference letter C) during a dry cleaning cycle thereof.

The perfuming element 10 comprises a perfuming substance P that is supported by a support element 11, which can gradually release the perfuming substance.

The support element 11 is made of a plastic material, for example, polyethylene.

In particular, the polyethylene used comprises a plurality of open micropores 110, for example exhibiting a diameter comprised between 50 and 5500 µm.

For example, the support element is made from a hydrophobic membrane having a high porosity, known by the commercial name of Accurel PP, marketed by the company Membrana Gmbh.

For example, the support element 11 is in the form of granules 111.

In particular, the polyethylene granules 111 used are microporous granules, with pores having a diameter comprised between 50 and 5500 µm.

For example the polyethylene granules are made by milling, granulation and/or fractioning of the high-porosity hydrophobic membrane known by the commercial name of Accurel PP.

For example, the granules exhibit a small-cylindrical shape, but they could also have different shapes.

For example, the granulometry of the support element is substantially comprised between 0.2 mm and 1 cm.

In practice, the perfuming substance enters by capillarity in the micropores of the support element 11 and the exit of the perfumed substance is slowed down, so as to have a gradual and slow release of the perfuming substance from the support element.

For example the release time of the perfuming substance is greater than an hour.

The perfuming substance comprises, for example, an essential oil.

The perfuming substance is in fluid form, more or less dense, and impregnates the internal volume of the support element 11.

The perfuming substance, for example, comprises a mixture of essential oils giving the desired perfuming.

The perfuming substance advantageously comprises a mixture of essential oils comprising among others at least one from among citronellyl nitrile, allyl cyclohexylpropionate, aldehyde c14, allyl amyl glycolate, vertocitral and dynascone pure.

In particular, the perfuming substance comprises a mixture of essential oils comprising one or more of the following essential oils: verdyl acetate, citral, cinnamic aldehyde, dihydromyrcenol, essence of Calabria bergamot, cinnamic alcohol, terpinyl acetate, citronellyl nitrile, geraniol, alpha hexyl cinnamic aldehyde, habanolide, pure aldehyde c-10, ethyl butyrate 10tri, isobornyl acetate, essence of Morocco rosemary, trimofix, orange crystals, benzyl acetate, distilled orange, essence of Brazil orange, mandarin, methyl anthranilate, anisic aldehyde, linalool, allyl cyclohexyl propionate, essence of Lavandin, essence of Litsea cubeba, styrallyl acetate, cis 3 hexen 10%, cis 3 hexenyl acetate, eucaliptol, ambroxan, mayol, agrumex, alpha amyl cinnamic aldehyde, aldehyde c 14, yara yara, citronellal, vanillin, phenylethyl alcohol, methyl benzoate, vertenex, citronellol, allyl amyl glycolate, aldehyde c 12 mna, vertocitral, aldehyde c 7 10%, dynascone pure, ethyl acetoacetate, benzoic aldehyde 10%, ethyl caprylate 10%, isoamyl acetate 10%, allyl heptylate and benzyl salicylate.

The mixture of essential oils advantageously comprises citronellyl nitrile in quantities comprised between 8 and 15 per thousand with respect to the weight of the mixture, allyl cyclohexylpropionate in quantities comprised between 12 and 20 per thousand with respect to the weight of the mixture, aldehyde c14 in quantities comprised between 20 and 30 per thousand with respect to the weight of the mixture, allyl amyl glycolate in quantities comprised between 7 and 18 per thousand with respect to the weight of the mixture, vertocitral in quantities comprised between 12 and 17 per thousand with respect to the weight of the mixture and dynascone pure in quantities comprised between 4 and 10 per thousand with respect to the weight of the mixture.

An example of a formulation of a perfuming substance is reported in the following table.

| Essential Oil | Gram Weight |
|---|---|
| verdyl acetate | 70 |
| citral | 7 |
| cinnamic aldehyde | 12 |
| dihydromyrcenol | 38 |
| ESS. CALABRIA BERGAMOT | 10 |
| cinnamic alcohol | 75 |
| terpinyl acetate | 87 |
| citronellyl nitrile | 8--15 |
| geraniol | 11 |
| alpha helxyl cinnamic aldehyde | 6 |
| HABANOLIDE | 12 |
| aldehyde c 10 pure | 19 |
| ethyl butyrate 10tri | 3 |
| isobornyl acetate | 11 |
| essence of Morocco rosemary | 5 |
| TRIMOFIX | 7 |
| ORANGE CRYSTALS | 28 |
| benzyl acetate | 5 |
| orange distillate | 25 |
| Essence of Brazil MANDARIN | 28 |
| methyl anthranilate | 24 |
| anisic aldehyde | 5 |
| linalool | 20 |
| ALLYL CYCLOHEXYL PROPIONATE | 12--20 |
| Essence of Lavandin | 6 |
| Essence of Litsea cubeba | 14 |
| styrallyl acetate | 18 |
| cis-3-hexenol 10% | 13 |
| cis-3 hexenyl acetate | 12 |
| eucalyptol | 14 |
| AMBROXAN | 4 |
| MAYOL | 6 |
| agrumex | 7 |
| alpha amylcinnamic aldehyde | 14 |
| aldehyde c 14 | 20-30 |
| yara yara | 70 |
| citronellal | 1 |
| vanillin | 10 |
| phenylethyl alcohol | 28 |
| methyl benzoate | 4 |
| vertenex | 20 |
| citronellol | 15 |
| allilamyl glycolate | 7--18 |
| aldehyde c 12 mna | 7 |
| vertocitral | 12--17 |
| aldehyde c 7 10% | 7 |
| dynascone pure | 4--10 |
| acetyl ethyl acetate | 2 |
| benzoic aldehyde 10% | 14 |
| 10% ethyl caprylate | 25 |
| isoamyl acetate 10% | 25 |
| allyl heptylate | 1 |
| benzyl salicylate | 85 |
| DPG | 7 |

To realise the perfuming element 10, the following process is followed.

A dosed quantity of support element 11 is placed in a stirrer, such as a mixer, and a second dosed quantity of perfuming substance is added thereto.

The perfuming substance is mixed with the support element for a predetermined time, e.g. 60 minutes.

The weight ratio between the first dosed quantity of support element 11 and the second dosed quantity of perfuming substance is for example 1:1.

During mixing, the perfuming substance is adsorbed internally of the micropores of the support element 11 and is trapped internally of the support element 11.

The perfuming element 10 is subsequently dosed and inserted in porous bags 12, which are able to release the fragrance of the perfuming substance while internally retaining the support element 11.

Each porous bag 12 for example can be made of a non-woven fabric or other porous material, such as fabric or a plastic support exhibiting micropores or the like.

Once each porous bag 12 is filled with a dose of perfuming element 10 it is closed, for example by heat sealing the open end and, for example, is packed inside a waterproof package 13, for example cellophane or double cellophane or another material able to contain internally thereof the fragrance of the perfuming substance until use thereof.

Each dose of perfuming element 10 present in the porous bag 12 comprises for example 10 grams of perfuming element 10, of which 5 grams of perfuming substance and 5 grams of support element 11.

Obviously the dose of perfuming element 10 in each porous perfuming bag 12 can be different from the one indicated above depending on needs and/or perfuming substance used.

A dry cleaning machine is shown schematically in figure 2 in the essential elements thereof and is generally indicated by reference number 20.

The machine 20 comprises a box-shaped body 21 equipped with a door that opens on a drum 22 that can receive one or more garments to be washed.

The drum 22 is rotatably associated for example to the box-shaped body 21, as known to the skilled person in the sector.

The machine 20 also comprises, for example, internally of the box body 21, one or more tanks 23 able to contain a cleaning solvent S.

For example, the cleaning solvent is selected from the group including perchlorethylene, liquid carbon dioxide and cyclic siloxane compounds.

Each tank 23 is served by a suction pump 24 that can withdraw the cleaning solvent from the tank 23 and introduce it into the drum 22 through a supply line 240.

The machine 20 also comprises a discharge pump 25 able to withdraw the cleaning solvent from the drum 22 and send it, via a discharge line 250, back to the tank 23.

The machine 20 can comprise, in addition, one or more filters 26, for example, able to intercept the discharge conduit 250 and/or located downstream of the drum 22, which can filter the washing solvent, retaining the fatty parts (grease, oil or otherwise) that the washing solvent has removed from the clothing.

The machine 20 further comprises a distiller element 27 which for example can intercept the discharge conduit 250, for example downstream of the filter 26, and is able to vaporize and recondense the solvent and separate the cleaning solvent from the oily and/or dirty parts removed from the garment.

The machine 20 comprises means for rotating (for example spinning) the drum 22 and, independently, means for drying the garments, for example a heating device, able to dry the garments by heating, thus removing the washing solvent therefrom.

In practice, the machine 20 can operate in closed cleaning cycles, in which the cleaning solvent is removed from the tank 23 and returned, at the end of the cleaning cycle, into the same tank for the next cleaning cycle.

The dry cleaning process of garments, according to the invention, includes steps of:
- locating at least a garment to be cleaned in the drum 22;
- adding into the drum 22, together with the garment to be cleaned, at least a dosed quantity of the perfuming element 10 of the garment into the drum 22; and
- introducing the cleaning solvent into the drum 22.

Once the drum 22 has been filled, it is rotated, so that the mechanical mixing action of the garment facilitates the stain-removing chemical action performed by the bath of cleaning solvent.

Subsequently, the cleaning solvent is extracted from the drum 22, while filtering and/or distilling the solvent that has come into contact with the garment to be washed.

In practice, the washing in the solvent bath is done cold, for example at ambient temperature (Tamb).

The granules 111 are in a substantially contracted conformation and, during washing, the perfuming substance is substantially prevented from exiting from the granules.

The perfuming substance including the perfuming element 10 is in each step of the cleaning released gradually and slowly by the support element 11 that prevents/limits the chemical attack by the solvent.

In practice, a majority of the perfuming substance remains inside of the support element 11 during the whole cleaning cycle, and when the cleaning solvent is extracted from the drum 22, the residual perfuming substance which is impregnated in the support element 11 can actively perfume the garment, imbuing it with the fragrance of the perfume which will remain in the fibres thereof.

In particular, when during the drying step of the garments the temperature in the drum 22 is raised above ambient temperature, and for example between 50°C and 60°C, the granules 111 expand into an expanded configuration, so as to enable and facilitate exit of the perfuming substance from the micropores 110 from the granules.

The perfuming element 10 is advantageously added in a quantity which respects the following proportion: between 5 and 100 grams of perfuming element for every 10 to 20 kg of clothing.

Satisfactory results have been observed, in terms of intensity and persistence, and the pleasantness of the fragrance imparted to the garments by the element 10, preferably for the following doses:
for a smaller amount of 20 kg of garments, 20 grams of perfuming element 10 (for example, two prepacked porous bags 12);
for an amount greater than 20 kg of garments, 30 grams of perfuming element 10 (for example three prepacked porous bags 12).

The solvent extracted from the drum 22 is then for example distilled and/or filtered, in order to be used again in a new cleaning cycle as described above.

Further, clothing can be dried, for example by centrifugation of the drum 22 or another known process of drying/solvent removal, such as heating.

At the end of the cleaning cycle, the support element 11 could be reused to adsorb fresh perfuming substance (operating as described above), so as to be re-bagged and used in a new cleaning cycle of cleaning and perfuming garments.

The invention as it is conceived is susceptible to numerous modifications and variants, all falling within the scope of the inventive concept.

Further, all the details can be replaced by other technically-equivalent elements.

In practice, the materials used, as well as the contingent shapes and dimensions, can be any according to requirements, without forsaking the scope of protection of the following claims.

## Claims

1. A perfuming element (10) for garments, usable in dry cleaning, based on solvents, which comprises a perfuming substance supported by a gradual-release support element (11) of the perfuming substance, **characterized in that** the support element (11) is made of polyethylene granules provided with micropores.

2. The perfuming element (10) of claim 1, wherein the micropores are open on the external surface of the granule.

3. The perfuming element (10) of claim 1 or 2, wherein the micropores exhibit a diameter comprised between 50 and 5500 µm.

4. The perfuming element (10) of claim 1, wherein the granulometry of the support element (11) is substantially comprised between 0.2 mm and 1 cm.

5. The perfuming element (10) of any one of the preceding claims, wherein the perfuming element (10) is contained internally of a porous bag (12).

6. The perfuming element (10) of claim 5, wherein the porous bag (12) is made of a non-woven fabric.

7. The perfuming element (10) of any one of the preceding claims, wherein the perfuming substance comprises at least an essential oil.

8. The perfuming element (10) of claim 7, wherein the perfuming substance comprises a mixture of essential oils comprising at least one from among citronellyl nitrile, allyl cyclohexylpropionate, aldehyde c14, allyl amyl glycolate, vertocitral and pure dynascone.

9. The perfuming element (10) of claim 8, wherein the mixture of essential oils comprises citronellyl nitrile in quantities comprised between 8 and 15 per thousand with respect to the weight of the mixture, allyl cyclohexylpropionate in quantities comprised between 12 and 20 per thousand with respect to the weight of the mixture, aldehyde c14 in quantities comprised between 20 and 30 per thousand with respect to the weight of the mixture, allyl amyl glycolate in quantities comprised between 7 and 18 per thousand with respect to the weight of the mixture, vertocitral in quantities comprised between 12 and 17 per thousand with respect to the weight of the mixture and dynascone pure in quantities comprised between 4 and 10 per thousand with respect to the weight of the mixture.

10. The perfuming element (10) of any one of the preceding claims, wherein the perfuming substance and the support element (11) are in a weight ratio of substantially 1:1.

11. A dry cleaning process of garments, which comprises steps of:
- locating at least a garment to be cleaned in a drum (22);
- introducing at least a liquid solvent into the cleaning drum (22);
- extracting the solvent from the cleaning drum (22) and
- filtering the solvent that has come into contact with the garment;
**characterised in that** it comprises a step of adding, into the cleaning drum, together with the garment to be cleaned, at least a dosed quantity of a perfuming element (10) of the garment according to any one of the preceding claims.

12. The process of claim 11, wherein the perfuming element (10) is added in a dose comprised between 5 and 100 grams per each 10-20 kilograms of garments.

13. The process of claim 11 or 12, **characterised in that** it comprises a step of distilling the solvent extracted from the cleaning drum (22).

14. The process of any one of claims from 11 to 13, wherein the solvent is a chemical solvent selected from a group including perchloroethylene, liquid carbon dioxide and cyclic siloxane compounds.

15. The process of any one of claims from 11 to 14, **characterised in that** it comprises a step of centrifuging the garment internally of the cleaning drum (22).

16. The process of any one of claims from 11 to 15, **characterised in that** it comprises a step of drying, by heating, the garment internally of the cleaning drum (22).

## Patentansprüche

1. Parfümierelement (10) für Kleidungsstücke, das in der auf Lösemitteln basierenden Trockenreinigung verwendbar ist und das eine Parfümiersubstanz umfasst, die von einem Trägerelement (11) der Parfümiersubstanz zur allmählichen Freisetzung getragen wird, **dadurch gekennzeichnet, dass** das Trägerelement (11) aus Polyethylengranulat besteht, das mit Mikroporen versehen ist.

2. Parfümierelement (10) nach Anspruch 1, wobei die Mikroporen an der Außenfläche des Granulats offen sind.

3. Parfümierelement (10) nach Anspruch 1 oder 2, wobei die Mikroporen einen Durchmesser zwischen 50 und 5500 µm aufweisen.

4. Parfümierelement (10) nach Anspruch 1, wobei die Granulometrie des Trägerelements (11) im Wesentlichen zwischen 0,2 mm und 1 cm liegt.

5. Parfümierelement (10) nach einem der vorhergehenden Ansprüche, wobei das Parfümierelement (10) im Inneren eines porösen Beutels (12) enthalten ist.

6. Parfümierelement (10) nach Anspruch 5, wobei der poröse Beutel (12) aus einem Vliesstoff besteht.

7. Parfümierelement (10) nach einem der vorhergehenden Ansprüche, wobei die Parfümiersubstanz mindestens ein etherisches Öl umfasst.

8. Parfümierelement (10) nach Anspruch 7, wobei die Parfümiersubstanz ein Gemisch aus etherischen Ölen umfasst, das mindestens eines der Folgenden umfasst: Citronellylnitril, Allylcyclohexylpropionat, Aldehyd c14, Allylamylglycolat, Vertocitral und reines Dynascone.

9. Parfümierelement (10) nach Anspruch 8, wobei das Gemisch aus etherischen Ölen Citronellylnitril in Mengen zwischen 8 und 15 pro tausend in Bezug auf das Gewicht des Gemischs, Allylcyclohexylpropionat in Mengen zwischen 12 und 20 pro tausend in Bezug auf das Gewicht des Gemischs, Aldehyd c14 in Mengen zwischen 20 und 30 pro tausend in Bezug auf das Gewicht des Gemischs, Allylamylglycolat in Mengen zwischen 7 und 18 pro tausend in Bezug auf das Gewicht des Gemischs, Vertocitral in Mengen zwischen 12 und 17 pro tausend in Bezug auf das Gewicht des Gemischs und reines Dynascone in Mengen zwischen 4 und 10 pro tausend in Bezug auf das Gewicht des Gemischs umfasst.

10. Parfümierelement (10) nach einem der vorhergehenden Ansprüche, wobei die Parfümiersubstanz und das Trägerelement (11) in einem Gewichtsverhältnis von im Wesentlichen 1:1 vorliegen.

11. Trockenreinigungsverfahren für Kleidungsstücke, folgende Schritte umfassend:
- Anordnen mindestens eines zu reinigenden Kleidungsstücks in einer Trommel (22),
- Einbringen mindestens eines flüssigen Lösemittels in die Reinigungstrommel (22),
- Extrahieren des Lösemittels aus der Reinigungstrommel (22) und
- Filtern des Lösemittels, das in Kontakt mit dem Lösemittel gekommen ist,
**dadurch gekennzeichnet, dass** es einen Schritt des Zugebens mindestens einer dosierten Menge eines Parfümierelements (10) für das Kleidungsstück nach einem der vorhergehenden Ansprüche in die Reinigungstrommel zusammen mit dem zu reinigenden Kleidungsstück umfasst.

12. Verfahren nach Anspruch 11, wobei das Parfümierelement (10) in einer Dosis zwischen 5 und 100 Gramm pro 10-20 Kilogramm Kleidungsstücke zugegeben wird.

13. Verfahren nach Anspruch 11 oder 12, **dadurch gekennzeichnet, dass** es einen Schritt des Destillierens des Lösemittels, das aus der Reinigungstrommel (22) extrahiert wird, umfasst.

14. Verfahren nach einem der Ansprüche 11 bis 13, wobei das Lösemittel ein chemisches Lösemittel ist, das aus einer Gruppe ausgewählt wird, die Perchlorethylen, flüssiges Kohlendioxid und cyclische Siloxanverbindungen umfasst.

15. Verfahren nach einem der Ansprüche 11 bis 14, **dadurch gekennzeichnet, dass** es einen Schritt des Schleuderns des Kleidungsstücks im Inneren der Reinigungstrommel (22) umfasst.

16. Verfahren nach einem der Ansprüche 11 bis 15, **dadurch gekennzeichnet, dass** es einen Schritt des Trocknens des Kleidungsstücks im Inneren der Reinigungstrommel (22) umfasst.

## Revendications

1. Élément parfumant (10) pour vêtements, utilisable dans le nettoyage à sec, à base de solvants, qui comprend une substance parfumante supportée par un élément de support de libération progressive (11) de la substance parfumante, **caractérisée en ce que** l'élément de support (11) est constitué de granules de polyéthylène munis de micropores.

2. Élément parfumant (10) selon la revendication 1, dans lequel les micropores sont ouverts sur la surface externe du granule.

3. Élément parfumant (10) selon la revendication 1 ou 2, dans lequel les micropores présentent un diamètre compris entre 50 et 5500 µm.

4. Élément parfumant (10) selon la revendication 1, dans lequel la granulométrie de l'élément de support (11) est sensiblement comprise entre 0,2 mm et 1 cm.

5. Élément parfumant (10) selon l'une quelconque des revendications précédentes, dans lequel l'élément parfumant (10) est contenu à l'intérieur d'un sac poreux (12).

6. Élément parfumant (10) selon la revendication 5, dans lequel le sac poreux (12) est constitué d'un tissu non-tissé.

7. Élément parfumant (10) selon l'une quelconque des revendications précédentes, dans lequel la substance parfumante comprend au moins une huile essentielle.

8. Élément parfumant (10) selon la revendication 7, dans lequel la substance parfumante comprend un mélange d'huiles essentielles comprenant au moins un parmi le nitrile de citronellyle, propionate de cyclohexyle allylique, l'aldéhyde c14, le glycolate d'amyle allylique, le vertocitral et le dynascone pur.

9. Élément parfumant (10) selon la revendication 8, dans lequel le mélange d'huiles essentielles comprend du nitrile de citronellyle dans des quantités comprises entre 8 et 15 pour mille par rapport au poids du mélange, du propionate de cyclohexyle allylique dans des quantités comprises entre 12 et 20 pour mille par rapport au poids du mélange, de l'aldéhyde c14 dans des quantités comprises entre 20 et 30 pour mille par rapport au poids du mélange, du glycolate d'amyle allylique dans des quantités comprises entre 7 et 18 pour mille par rapport au poids du mélange, du vertocitral dans des quantités comprises entre 12 et 17 pour mille par rapport au poids du mélange et du dynascone pur dans des quantités comprises entre 4 et 10 pour mille par rapport au poids du mélange.

10. Élément parfumant (10) selon l'une quelconque des revendications précédentes, dans lequel la substance parfumante et l'élément de support (11) sont dans un rapport de poids de sensiblement 1:1.

11. Procédé de nettoyage à sec de vêtements, qui comprend les étapes suivantes :
- la mise en place d'au moins un vêtement à nettoyer dans un tambour (22) ;
- l'introduction d'au moins un solvant liquide dans le tambour de nettoyage (22) ;
- l'extraction du solvant du tambour de nettoyage (22) et
- le filtrage du solvant qui a été mis en contact avec le vêtement ;
**caractérisé en ce qu'**il comprend une étape d'ajout, dans le tambour de nettoyage, conjointement au vêtement à nettoyer, d'au moins une quantité dosée d'un élément parfumant (10) du vêtement selon l'une quelconque des revendications précédentes.

12. Procédé selon la revendication 11, dans lequel l'élément parfumant (10) est ajouté dans une dose comprise entre 5 et 100 grammes tous les 10-20 kilogrammes de vêtements.

13. Procédé selon la revendication 11 ou 12, **caractérisé en ce qu'**il comprend une étape de distillation du solvant extrait du tambour de nettoyage (22).

14. Procédé selon l'une quelconque des revendications 11 à 13, dans lequel le solvant est un solvant chimique sélectionné à partir d'un groupe comprenant le perchloroéthylène, le dioxyde de carbone liquide et des composés de siloxanes cycliques.

15. Procédé selon l'une quelconque des revendications 11 à 14, **caractérisé en ce qu'**il comprend une étape de centrifugation du vêtement à l'intérieur du tambour de nettoyage (22).

16. Procédé selon l'une quelconque des revendications 11 à 15, **caractérisé en ce qu'**il comprend une étape de séchage, par chauffage, du vêtement à l'intérieur du tambour de nettoyage (22).
